# EUROPEAN PATENT SPECIFICATION

(11) **EP 2 936 152 B1**
(45) Date of publication and mention of the grant of the patent: **22.04.2020**
(21) Application number: 13812049.8
(22) Date of filing: 23.12.2013
(51) Int. Cl.: G01N 33/53, G01N 33/68

(54) **IMMUNOASSAY**
IMMUNOASSAY
IMMUNO-ESSAI

(30) Priority: 21.12.2012 GB 201223256
(43) Date of publication of application: 28.10.2015
(73) Proprietor: LumiraDx UK Limited, London SE1 2AQ (GB)
(72) Inventor: MAZZOLENI, Giorgio, London SW7 2AZ (GB); BALDRACCHINI, Francesca, London SW7 2AZ (GB); MACCARI, Mauro, London SW7 2AZ (GB); GRÖNLUND, Hans Anders Conrad, London SW7 2AZ (GB)
(74) Representative: Stratagem IPM Limited
(86) International application number: PCT/GB2013/053416
(87) International publication number: WO 2014/096871

(56) References cited:
- MASTRANDREA F ET AL: "IgE responses to Dermatophagoides pteronyssinus native major allergens Der p 1 and Der p 2 during long-term specific immunotherapy", ALLERGY, WILEY-BLACKWELL PUBLISHING LTD, UNITED KINGDOM, vol. 52, no. 11, 1 November 1997 (1997-11-01), pages 1115-1119, XP009163814, ISSN: 0105-4538
- STEWART, G.A., ET AL: "Isotype specific immunoglobulin response to the house dust mite Dermatophagoides pteronyssinus and the purified allergen Der p1 in asthmatic and control subjects from the Eastern Highlands of Paua New Guinea", CLINICAL ALLERGY, vol. 18, 1988, pages 235-243, XP009175560,
- M. BRONNERT ET AL: "Component-resolved diagnosis with commercially available D. pteronyssinus Der p 1, Der p 2 and Der p 10: relevant markers for house dust mite allergy", CLINICAL & EXPERIMENTAL ALLERGY, vol. 42, no. 9, 1 September 2012 (2012-09-01), pages 1406-1415, XP055097322, ISSN: 0954-7894, DOI: 10.1111/j.1365-2222.2012.04035.x
- DIEGO O. MIRANDA ET AL: "Serum and Salivary IgE, IgA, and Antibodies to Dermatophagoides pteronyssinus and Its Major Allergens, Der p1 and Der p2, in Allergic and Nonallergic Children", CLINICAL AND DEVELOPMENTAL IMMUNOLOGY, vol. 163, no. 5, 1 January 2011 (2011-01-01), pages 2944-11, XP055097321, ISSN: 1740-2522, DOI: 10.1111/j.1365-2222.1996.tb00055.x
- GIOVANNI MELIOLI ET AL: "The ImmunoCAP ISAC molecular allergology approach in adult multi-sensitized Italian patients with respiratory symptoms", CLINICAL BIOCHEMISTRY, ELSEVIER INC, US, CA, vol. 44, no. 12, 5 May 2011 (2011-05-05), pages 1005-1011, XP028239278, ISSN: 0009-9120, DOI: 10.1016/J.CLINBIOCHEM.2011.05.007 [retrieved on 2011-05-19]
- HALES ET AL: "IgE and IgG anti-house dust mite specificities in allergic disease", JOURNAL OF ALLERGY AND CLINICAL IMMUNOLOGY, ELSEVIER, AMSTERDAM, NL, vol. 118, no. 2, 1 August 2006 (2006-08-01), pages 361-367, XP005587352, ISSN: 0091-6749, DOI: 10.1016/J.JACI.2006.04.001

## Description

The present invention relates to the field of immunoassays, which detect and/or quantify antigen-specific immunoglobulin, and more particularly to immunoassays that are used in the diagnosis of allergic conditions or diseases.

### Background to the invention

Allergic disease has increased in prevalence over the last few decades, most notably in the more developed areas of the world. An allergy is an immune response to substances that are usually not harmful in the environment. The body identifies the substance as a threat and produces an inappropriate, exaggerated response to it. The substance that causes a reaction is called an allergen, and these can include substances such as pollen, dust mites, animal dander, mould spores, latex, drugs, insect venom, and foods such as nuts and egg.

Allergies are a major health problem and cause significant economic losses, and thus correct diagnosis and treatment are key.

Individuals that suffer with allergies present various clinical symptoms of the disease. These include: respiratory (such as sneezing, wheezing and acute bronchospasm), cardiovascular (such as hypotension), cutaneous (such as swelling and hives), gastrointestinal (for example, vomiting and diarrhoea) or generalised symptoms (including anaphylaxis). Common allergic disorders include asthma, eczema and hay fever. Once a patient presents in a primary care setting with symptoms that involve irritation and inflammation, a clinician usually undertakes further tests in order to determine which allergens are causing the allergic response. It is the combination of clinical symptoms and the presence of immunoglobulins to one or more allergens that leads to a diagnosis of allergy.

In order to make a diagnosis of an allergy, clinicians can use skin-prick tests. The skin is pricked with a tiny amount of the suspected allergen to see if there is a reaction. If there is, the skin around the prick will swell and become red and itchy, the typical signs of an immune response to that allergen. However, skin-prick tests are not always desirable, since most notably they can be uncomfortable, and involve a large area of skin for comprehensive testing. Further, if the individual has a life-threatening reaction to any allergens, these tests are not recommended. If an individual is taking antihistamines or tri-cyclic antidepressives, the skin prick tests may not be reliable.

The antigenicity of a given substance may be derived from a number of single antigens within that substance. Antigenic components ("components") may be proteins, polysaccharides or any other entity capable of stimulating an immune response, containing an epitope. For example, if the substance is dust mite (*Dermatophagoides pteronyssinus*)*,* this contains all the molecules to sustain the organism's life, but it also contains allergenic fractions consisting of antigenic components like Der p 1 and Der p 2. Der p 1 and Der p 2 in particular are major allergenic components of the natural dust mite extract. Generally, the lower the fraction of the allergenic molecules in a given extract, the lower is the sensitivity an assay based on the extract.

Immunoglobulin E (IgE) is a type of antibody that plays a major role in allergic diseases. IgE binds to allergens and triggers the release of substances from mast cells that can cause inflammation. In susceptible people, B cells produce allergen-specific IgE in response to the initial exposure to the allergen. This IgE then circulates in the blood; binds to mast cells and upon subsequent exposure to the same allergen, a cascade of allergic reaction can begin. Specific IgE has thus become an established marker for allergic disease.

Immunoassays are available to determine the levels of IgE specific for a particular allergen. These are foremost blood tests that evaluate the presence and possibly quantify the various allergen-specific IgE antibodies in sensitised subjects.

Immunoassays are methods used to assay for the presence of an antigen or a particular antigen-specific antibody in a given sample. The detection of the antigen bound to the antigen-specific antibody (i.e. an immune-complex) can be achieved by incubating the sample over a particular antigen. Subsequently the presence of antigen-specific antibodies can be visualised using various techniques. In general, immunoassays require the immobilisation of an antigen on a solid support. Types of immunoassays include ELISAs (enzyme linked immunosorbent assay) and RASTs (radioallergosorbent test, including the fluorescence based ImmunoCAP™ assay format from Phadia AB).

Allergy *in-vitro* diagnostic immunoassays are being used as an alternative to skin testing, since they are more convenient for the individual, as they need only to provide a blood sample. However, it has been found in a report (Bernstein et al, Allergy Diagnostic testing: an updated practice parameter. Part 2. Ann Allergy Asthma Immunol 2008 Mar; 100 (3 suppl) S66-S121) that the sensitivity of blood testing can be up to 25-30% lower than skin testing.

Present clinical practice for blood testing of individuals with possible allergies is to test generally for the presence of immunoglobulins in a sample, such as blood, which bind to a substance. That substance is an allergenic or antigenic substance, such as peanut, dog dander or birch pollen. It may be presented in an immunoassay as a natural extract of the substance, or a component isolated from the natural extract, but never both in parallel. Such assays aid the clinician in the decision "allergic to peanut" or "allergic to birch pollen" but no further information. Further, each allergic patient has a unique allergen-specific IgE profile. If individual components alone are used, an allergy can be missed as a particular antigenic component may be missing, or not yet identified. Allergies are complex, since most substances contain at least one antigenic component. Indeed, some cross reactivity between antigenic components is known. For example, allergens Bet v 1 from birch pollen and Ara h 8 from peanut are known to be cross-reactive, and an immunoglobulin specific to one of these may also bind to the other.

Only in limited circumstances are individuals subjected to a second blood test, which seeks to resolve the components of the substance to which the individual possesses immunoglobulins. This can be critical, since the presence of certain component-specific immunoglobulins can indicate potentially anaphylactic reaction to the substance and others not. For example, a birch pollen allergic patient with IgE antibodies toward Bet v 1 who is eating peanuts (containing Ara h 8) may experience itching in the mouth (oral symptoms) due to the presence of a cross-reacting antibodies but not anaphylactic reactions.

The present invention seeks to improve the sensitivity and quality of immunoassays for allergy, and indeed immunoassays for any substance. It provides a more sensitive immunoassay for quantifying specific immunoglobulin levels in a test sample. In improving the sensitivity and quality of the immunoassay, the limit of detection of specific immunoglobulin in a test sample is also improved. Furthermore, an individual can receive a more specific diagnosis that allows for precise management of clinical symptoms.

### Summary of the invention

It is an object of the invention to provide an improved immunoassay which has increased sensitivity and quality. Specifically, the present invention is a method of detecting and/or quantifying substance-specific immunoglobulin levels. The invention further relates to a system that enables such detection and quantification. The method can be used to diagnose sensitisation to an allergen or an allergy in an individual, particularly when the symptoms of that patient are taken into consideration.

More particularly, it is an object of the invention to provide an improved immunoassay that provides a more sensitive diagnostic tool and which advantageously can even be directly used in a primary care setting.

These objects are achieved by the novel provision of a plurality of forms for each substance of interest. For example, the substance of interest can be derived from peanut. More than one substance can be included if required, and thus each tested substance is provided in a plurality of forms. For example, the substance is provided as a natural extract and one or more antigenic components derived from the substance. As an example, this could be natural extract of peanut as one form and antigenic components Ara h 1, Ara h 2 and Ara h 5. These discrete substance forms are immobilised separately on the solid support, if a solid support is used. Thus, each of the forms is assessed separately in an immunoassay, and a distinct result is obtained for each form. Thus, for each tested substance, a plurality of forms produces a plurality of results. In the example in relation to peanut, 4 separate results would be obtained, one for the natural extract and one for each of the components. When the results are analysed, one or more of the substance forms can be combined, for example, the results from two or more of the antigenic components can be summed. From these results, the levels of substance-specific immunoglobulin in a sample can be quantified. It is the system of analysis of these results that allows a clinician to assess more precisely if an individual has specific immunoglobulin that bind to the tested substance. For the individual antigenic component(s) present in the method, the result will demonstrate the presence and/or level of antigenic component-specific immunoglobulin.

It is important to note that the positivity of an *in vitro* diagnostic test can only be established when a sufficient amounts of both substance-specific immunoglobulin and said substance are present in the assay. In particular, if not enough or low-quality substance is present in the assay, the substance-specific immunoglobulin will not be able to sufficiently bind to said substance, which results in a poor performance of the assay (e.g. low sensitivity).

Thus, in a first aspect the present invention provides:
A method for detecting the presence or absence of substance-specific immunoglobulin in a sample, said method comprising contacting said sample with a plurality of forms of said substance substantially simultaneously and determining for each form separately whether an immune-complex is formed, wherein at least one form is a natural extract, modified by removal of one or more antigenic components.

It is preferred that at least one form is an antigenic component or a combination of antigenic components.

It is preferred that said method can be used for the quantification of levels of substance-specific immunoglobulins in a sample. It is further preferred that said method can alternatively or additionally be used for the quantification of levels of antigenic component-specific immunoglobulin. The method of the invention can thus include one or more steps to quantify the levels of substance and/or antigenic component-specific immunoglobulin.

In another preferred aspect of the invention, the method may be used to enable the diagnosis of an allergy in an individual. The individual supplies the sample used in the method. The method of the invention can thus include one or more steps to determine the sensitisation status of an individual. Alternatively or additionally, the method can be used to determine the substance-specific or antigenic component-specific immunoglobulin status of an individual.

As used herein, sensitisation means that the patient has previously been exposed to a substance, and immunoglobulins have been raised against that substance. Further exposure may result in allergic symptoms as discussed previously.

In a second aspect, there is provided a method for the quantification of levels of an substance-specific immunoglobulin in a sample, said method comprising contacting said sample with a plurality of forms of said substance and determining the level of reactivity of said immunoglobulin to said substance forms, wherein at least one form is a modified natural extract.

In a third aspect there is provided a method for enhancing the binding capacity of a substance, comprising the selective removal of one or more components. It is preferred that the substance is prepared as a natural extract. The binding capacity relates to the ability of substance-specific binding members such as immunoglobulins to bind to the substance. Said substance (described herein as a modified or depleted substance) can be used in any method, array or kit of the invention.

In a fourth aspect, there is provided an array comprising a plurality of forms of a substance provided at discrete locations, wherein at least one form is a natural extract, modified by removal of one or more antigenic components, of said substance.

Said array can be used in any of the methods or kits of the invention.

It is preferred that at least one antigenic component or a combination of antigenic components is provided as a form of said substance. Thus, 1 or more forms may be an antigenic component or antigenic component mix.

In a fifth aspect, there is provided a kit comprising an array which comprises a plurality of forms of a substance provided at discrete locations as described above and one or more detection binding agents.

In a sixth aspect, there is provided a method to determine the substance - specific immunoglobulin status of an individual comprising contacting a sample from said individual substantially simultaneously with a plurality of forms of said substance, wherein at least one form is a natural extract, modified by removal of one or more antigenic components, and determining whether an immune complex is formed.

In a seventh aspect, there is provided a method for the diagnosis of an allergy to a substance in an individual, said method comprising contacting a sample from said individual substantially simultaneously with a plurality of forms of a substance, wherein at least one form is a natural extract, and the presence of an immune complex with one or more of the forms indicates the presence of a sensitisation or and/or allergy to that substance.

### Brief description of figures

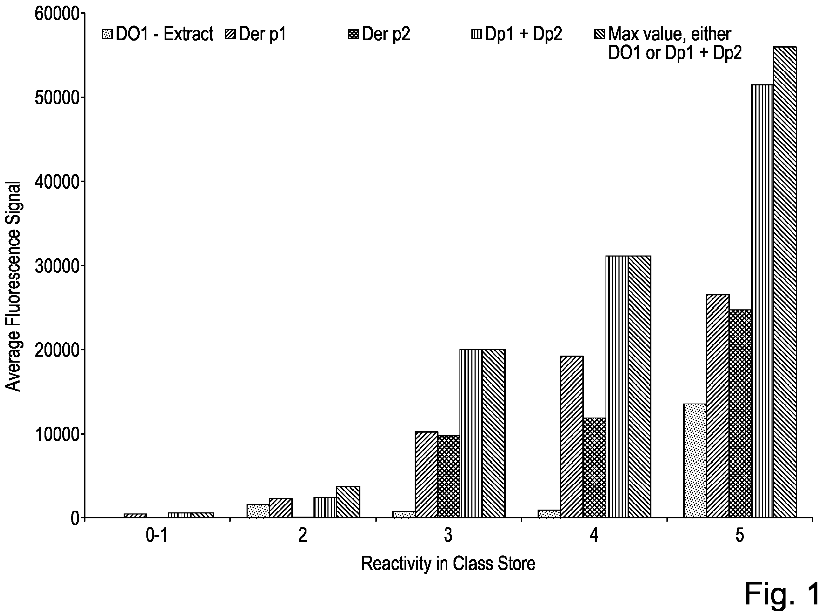

Figure 1: Figure 1 shows the results from Example 1. It is a bar chart showing the allergen-specific immunoglobulin (IgE) concentration (Reactivity in Class Score) versus average fluorescent signal for Dust Mite Extract (natural extract), Der p 1 and Der p 2 (both components).

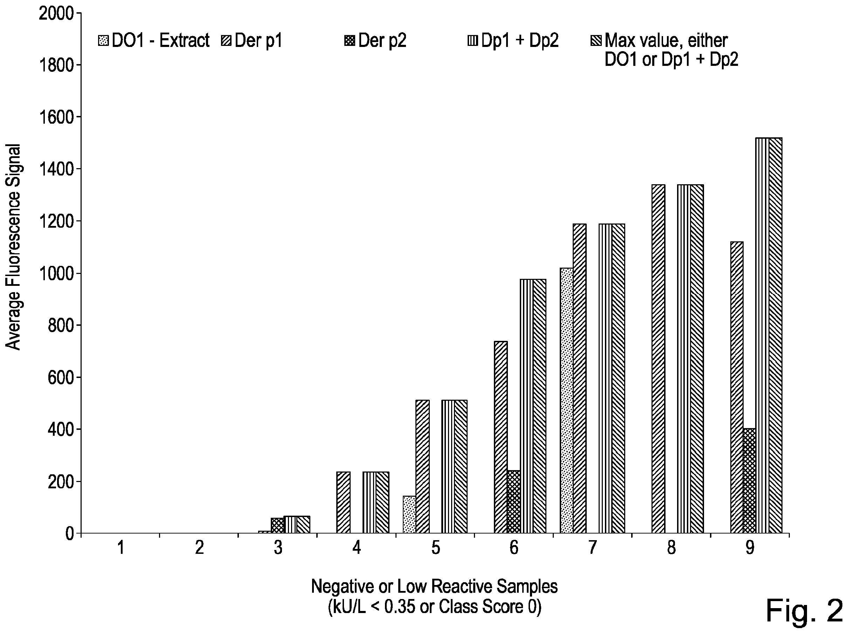

Figure 2 is a further bar chart showing results from Example 1, and is similar to figure 1, except is shows negative or low reactive samples (allergen-specific immunoglobulin concentration < 0.35 kU/L). The bar chart show how both sensitivity (i.e. higher reactivity) and specificity (i.e. negative samples remain negative) can be improved by using the fluorescence signal derived from either the single antigen/allergen (e.g. natural extract) or a combination of them (e.g. Natural altered extract and/or single antigens)

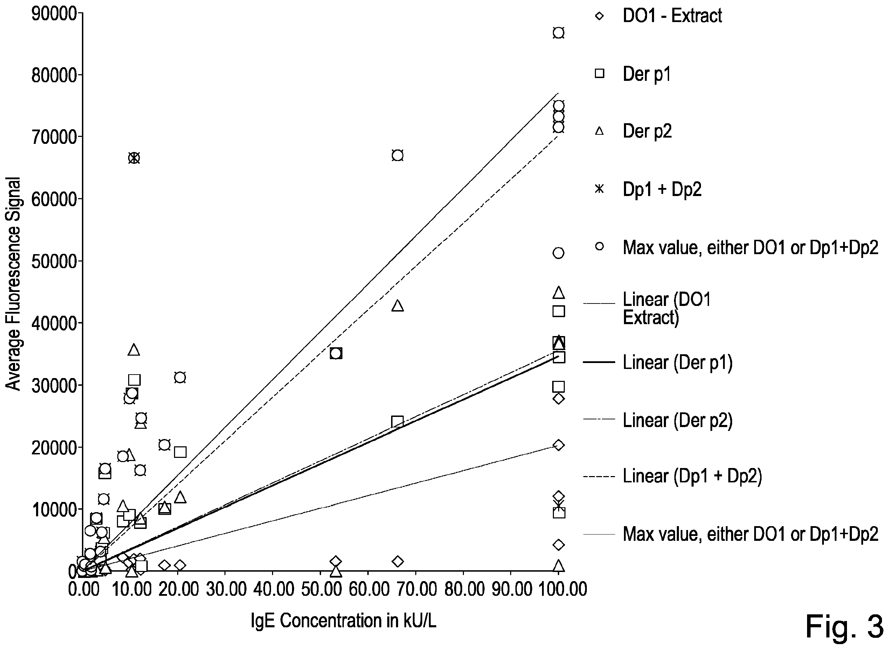

Figure 3: shows the results from Example 1 plotted on a graph of IgE concentration versus Average fluorescent signal. To help the understanding of the plot, a trendline for each condition was superimposed to the data (e.g. higher slope value indicates higher assay sensitivity).

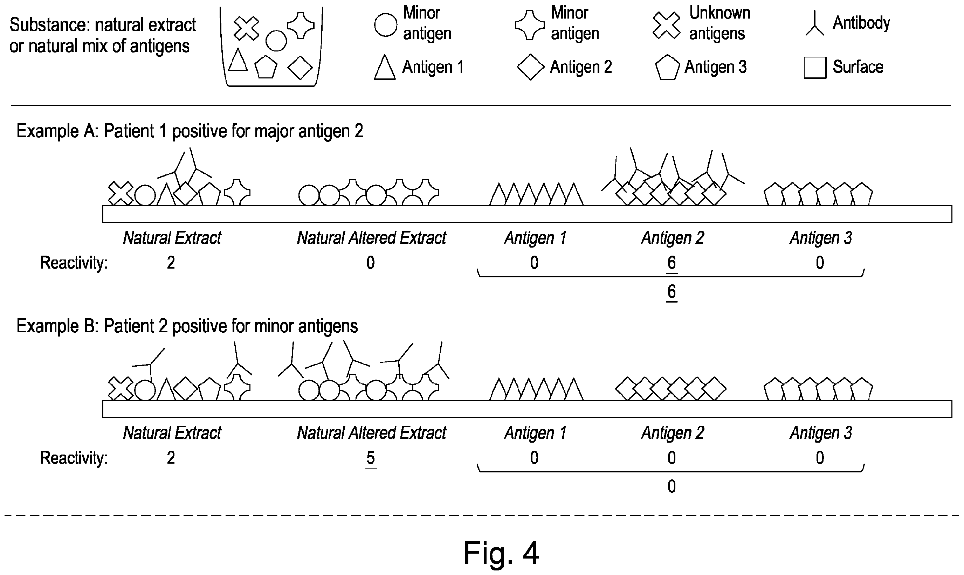

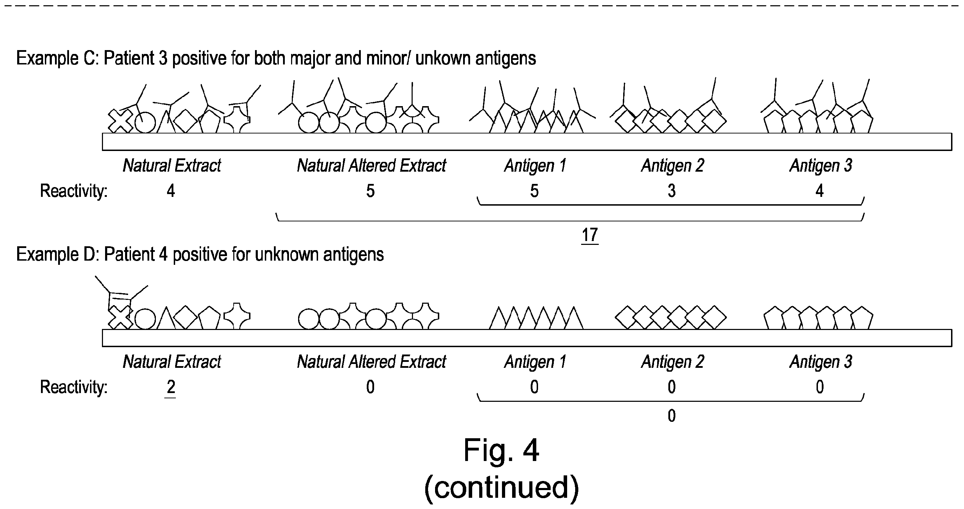

Figure 4: demonstrates pictorially the core of the invention that a plurality of forms of a substance are presented to the sample, including the natural extract. The natural extract is shown as a natural mix of antigens (antigenic components). Depicted are two minor antigens, an unknown antigen, major antigens, antibodies and the surface of the array. Example A shows an array for Patient 1 positive for major antigen 2. Example B shows an array for Patient 2 positive for minor antigens. Example C shows patient 3 positive for both major and minor/unknown antigens and Example D depicts Patient 4 who is positive for unknown antigens.

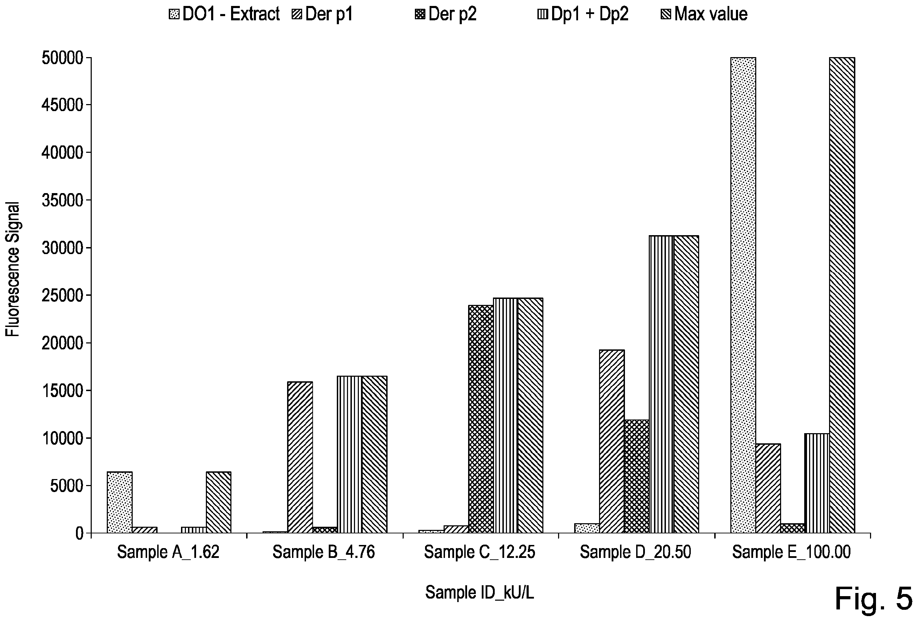

Figure 5: show select data from Example 1 that highlights the utility of the invention. 5 samples are presented where sensitivity to Dust Mite would not have been detected if natural extract or individual components had been used in isolation. The data is presented as a bar chart of sample versus fluorescent signal.

### Detailed description of the invention

The inventors have discovered that it is possible to improve the sensitivity and quality of an immunoassay by providing a substance in a plurality of discrete forms including at least one natural extract of the substance, the natural extract being modified by removal of one or more antigenic components. The forms of the substance may also include antigenic components of the substance (such as known allergens), either individually or in combination with one or more other antigenic components. The reactivity data from these discrete substance forms is then analysed to provide information on whether an individual (from whom the sample is taken) possesses substance-specific immunoglobulin. The substance forms are immobilized separately on the solid support if a support is used. The method of the invention also allows for the detection and/or quantification of antigenic component-specific immunoglobulins, since the individual components generally present one type of antigen or allergen in the assay to the sample, and these individual results can also be read or determined.

It will be understood throughout that where an antigenic component is discussed, this component may be an allergen or antigen.

The present invention can be used in an array, or other suitable format, where just one substance is being tested, or where multiple (2 or more) substances are being tested. Where multiple substances are being tested, one or more of these substances may be present in a plurality of forms. Thus, it is possible that all of the tested substances are present in a plurality of forms, or some (10, 20, 30, 40, 50, 60, 70, 80 or 90%) of the substances are present in a plurality of forms. It is an object of the invention that in the case of multiple substances in an immunoassay, at least one of these substances is present in a plurality of forms with at least one of these forms being a natural extract of the substance. If multiple substances are tested, there may be any number between 2 and 10,000, more particularly 2 to 1000 substances tested in one immunoassay, more particularly 2 to 500, even more particularly 2 to 250 separate substances used.

It is preferred that the methods of the invention are performed on a single array format, with all of the substance forms being located on a single chip or plate, for example.

There is thus provided a method for detecting the presence or absence of substance-specific immunoglobulin in a sample, said method comprising contacting said sample substantially simultaneously with a plurality of forms of said substance and determining whether an immune-complex is formed, wherein at least one form is a natural extract, modified by removal of one or more antigenic components.

It is preferred that said method allows for the quantification of the level of substance-specific immunoglobulin in a sample. Quantification relates to the determination or expression of the quantity or level of substance-specific immunoglobulin. It is preferred that the level of substance-specific immunoglobulin is quantified for each form of the substance. The level or quantity of substance-specific immunoglobulin is measured according to the detection method used.

It is preferred that one or more of the substance forms is an antigenic component of said substance. If antigenic components are used in the method of the invention, the method allows for detection and quantification of antigenic component-specific immunoglobulin in a sample.

In a second aspect, there is provided a method for the quantification of levels of an substance-specific immunoglobulin in a sample, said method comprising contacting said sample with a plurality of forms of said substance and determining the level of reactivity of said immunoglobulin to said substance forms, wherein one form is a natural extract, modified by removal of one or more antigenic components.

The methods of the invention require the presence of a plurality of forms of a substance, at least one of which is a natural extract, modified by removal of one or more antigenic components. As used herein, a substance is an entity, physical matter or material. It is preferred that the substance is natural in origin, but can be presented in modified form (i.e. latex) or be entirely synthetic (i.e. perfume). For the purposes of the invention, the substance is antigenic; it is capable of interacting with the immune system and provoking an immune response. The antigenicity of the substance may be derived from a number of single antigens within that substance. Without being limited, the following are examples of substances that could be used in the present invention:
Eggs, shellfish, fish, peanuts, tree nuts, soybeans, celery, mustard, sesame, milk, lupin, garlic, oats, wheat, tree pollen (for example, oak, ash, birch, elm, alder, cedar, hazelnut, willow, olive, hornbeam), grass pollen (for example, ryegrass and timothy), weed pollen (ragweed, nettle, mugwort, sorrel and goosefoot), feathers, cat, dog dander, cockroach, dust mite, insect venom such as bee venom, rodent (in particular rodent urine), mould spores, bacteria, viruses, parasites, latex, perfume, and self proteins such as myelin.

The present inventors realised that the trend to move towards testing only single components from substances when testing for conditions such as allergy in most cases has led to a decrease in the sensitivity in the detection of allergies. This is due to the loss of testing against antigenic components that either have not yet been identified or they are present in low amounts in the extract. Furthermore, this is also the case if a substance is presented as a natural extract "spiked" with a known antigenic component, since, in this case the remaining antigens may be diluted or masked in the process. This decrease in sensitivity could lead to false negative results for an individual, particularly if they are allergic to an uncharacterised antigen in the substance. The present invention seeks to improve the sensitivity (e.g. better detection limit) of substance-specific immunoglobulin(s).

It is preferred that the substance forms are bound to a suitable support, such as a solid surface for the purposes of this invention. Each of the forms are bound separately, at known locations. These locations may be in close proximity or at distance. The provision of a solid surface or support is discussed further below.

When methods of the invention are performed, it is preferred that each of the forms of the substance are contacted with a sample substantially simultaneously. This means that each of the forms are contacted with a sample at essentially the same time, or in parallel.

A sample, as used herein, is a specimen taken from an individual. The sample is preferably a bodily fluid, including, but not limited to blood, plasma, serum, lymph, urine or saliva.

An individual denotes a subject, regardless of whether they are likely to possess immunoglobulins to the tested substance(s), for example, whether or not they have an allergic disease. For the purposes of the invention, the individual is preferably a human, but could be an animal, most preferably a mammal.

An immunoglobulin is one of a class of gamma globulin proteins that are present in the serum, lymph and cells of the immune system, that function as antibodies. Antibodies of the main types (IgA, IgD, IgE, IgG and IgM) are included within this definition. These proteins are produced in response to the presence of an antigenic substance, and are specific for that substance. As used herein, the immunoassay is to detect a substance-specific immunoglobulin.

Immunoglobulin E (IgE) directed towards a specific allergen is not normally detected in a sample from an individual and is only produced when an individual becomes sensitised to that allergen. IgE which is directed towards a particular allergen and that will only react with that allergen is generally known as a specific IgE (slgE). An individual may have specific IgE to more than one allergen. This principle also applies to other antibodies and antigens, the antibodies produced are specific for the antigen.

An immune complex is formed from the integral binding of an immunoglobulin such as an antibody to an antigen.

Reactivity is used to refer to the level of binding between a particular antibody and its ligand to form an immune complex. In the context of the present invention the immunoglobulin(s) are specific antibodies and the ligands are specific antigens. In the case of allergic disease, generally a specific IgE will bind to a specific allergen. The level of reactivity may be defined in IU/ml or alternatively in terms of ranges which may be assigned Class Score values. For example, Class 0 being less than 0.35 IU/ml; Class 1 being from 0.35 to 0.7 IU/ml; Class 2 being from 0.71 to 3.5 IU/ml; Class 3 being from 3.51 to 17.5 IU/ml; Class 4 being from 17.51 to 50 IU/ml and Class 5 being from 50.01 to 100 IU/ml.

Alternatively, the antibody reactivity to a particular antigen may be considered to be either positive/present or negative/absent, for example below a threshold the response is deemed negative/absent and above the threshold the response is positive/present. Generally a level of > (greater than) 0.35 IU/mL indicates a positive result, in other words that a specific antibody has bound to its ligand. It is the antibody-antigen (immune) complexes formed as a result of interaction between a specific antibody in a sample and its ligand, in this case an antigen, that is measured with a suitable assay.

An antigen is an entity that is capable of causing an immune response. Generally an antigen is an entity foreign to the body, but occasionally, the antigen can be native to the individual, and thus the immune response is an autoimmune response. An epitope is a part of an antigen that the immune system recognises. The epitope may only be a few residues in length and these may be sequential or spatial in arrangement. Larger antigens can contain more than one epitope. As used herein, a substance can contain 1 or more antigens, named herein as antigenic components.

In the context of this invention, the term allergen means a specific type of antigen that can trigger an allergic response which is mediated by IgE antibody.

The method and preparations of this invention are directed to "forms of substance" or "substance forms". This denotes that the substance is present in the "form" of a natural preparation or extract (for example, dust mite extract) and also antigenic components isolated from the natural preparation/extract (following the same example, components Der p 1 and Der p 2). It will be understood by those skilled in the art that the antigenic component "forms" may either be isolated from the natural source, or produced using recombinant genetic techniques. It is preferred aspect of the invention that the substance tested in the method is present in the natural extract form, modified by removal of one or more antigenic components, and one or more antigenic component forms.

Thus, a plurality of forms of a substance can be a natural extract, modified by removal of one or more antigenic components, and one or more antigenic components. More than one natural extract, modified by removal of one or more antigenic components can be provided if desired. Any number of substance forms may be provided, preferably 2 or more, but 3, 4, 5, 6, 7, 8, 9, 10 or more substance forms can be provided. Each form is distinct, although it should be appreciated that most methods are run in at least duplicate in order to validate the results obtained. The substance-specific immunoglobulin from the sample are able to bind to any of the forms which contain the epitope for which they are specific.

The present invention includes a plurality of forms of a substance, each of which is distinct. At least one of these forms is a natural extract, modified by removal of one or more antigenic components.

In a further embodiment, at least one form of the substance is an antigenic component of that substance. The antigenic component is described further below, but can be isolated from the substance or produced recombinantly. Thus, 1, 2, 3, 4, 5, 6, 7, 8, 9 or more forms may be an antigenic component. The antigenic component can be presented individually (each antigenic component being a substance form) or in combination with 1 or more (1, 2, 3, 4, 5, 6, 7, 8, 9 or more) other antigenic components as a singular substance form. For example, one of the substance forms could be a combination of 2 antigenic components, such as Der p 1 and Der p 2. Both of these antigenic components are known allergens. This combination would be presented as one form of the substance, and could include 2 or more antigenic components.

It will be understood that if components are utilised in the method of the invention, then component-specific immunoglobulin from the sample will be able to bind. The presence of individual or combined antigenic components allows a greater resolution of the allergic profile. It is preferred that the component is antigenic or allergenic.

In one aspect, the present invention is a method for detecting the presence or absence of substance-specific immunoglobulin in a sample, said method comprising contacting said sample with a plurality of forms of said substance substantially simultaneously and determining whether an immune-complex is formed, wherein one form is a natural extract, modified by removal of one or more antigenic components, and at least one form is an antigenic component of said substance.

The method of the present invention may be used to help diagnose allergy to a substance in an individual, based upon the presence and/or level of substance-specific immunoglobulins in the sample. The diagnosis is typically made in conjunction with the clinical history of an individual, such as symptoms including for example, sneezing; wheezing; breathing difficulties; sinus pain; runny nose; coughing; nettle rash (hives); swelling; itchy or irritated eyes, ears, lips, throat and/or palate; and/or sickness, vomiting and/or diarrhoea.

It should be noted that the antigenic components used as one form of the substance in the methods and arrays of the invention may also be present in the natural extract form. This presence is part of the normal composition of the natural extract; it has in no way been added or spiked in the natural extract. The present invention does not read onto natural extracts that have been spiked with antigenic component.

The natural extract is a preparation of the substance. For example, it can be a preparation of dust mite, peanut, or animal dander. It comes from the natural source of the antigen, which includes synthetic compounds if these are capable of producing an immune response. Natural extracts can be prepared according to any suitable method, including aqueous extraction of the substance, obtained from natural sources. The product method is ideally intended to be compatible with the physiological conditions to which the substance is exposed upon contact with the individual. This would result in an unmodified natural extract of the substance.

The natural extract is treated to remove one or more selected components, resulting in modified or altered natural extract The term natural extract as used herein also extends to natural extracts treated to remove one or more selected components, and thus encompasses modified or altered natural extracts. It is a preferred aspect of this invention that the natural extract is treated in order to remove antigenic components that are also presented in the immunoassay/method. Such a modified natural substance can also be referred to as a "depleted" natural extract, since components have been removed. Thus, if one of the substance forms in the assay is an antigenic component A, the natural extract form can be treated to remove antigenic component A, leaving the remaining components intact. This helps achieve higher sensitivity, since the minor components in the natural extracts are not diluted by the presence of a major antigenic component. For example, for determining whether an individual is allergic to dust mite, the following forms might be used in an immunoassay:
Natural dust mite extract minus Der p 1 and Der p 2
Der p 1 (antigenic component)
Der p 2 (antigenic component)

Separating out known antigenic component forms from the natural extract allows for a better sensitivity of the assay, since it is likely that an individual will possess antigen-specific antibodies that do not bind to the isolated components, but rather to a minor component of the natural extract form. The component resolution is increased and also the sensitivity. Alternatively or additionally, the natural extract can be treated to remove antigenic components that are cross reactive. Several known allergens share a common polypeptide or polysaccharide sequence, or peptide or sugar structures against which antibodies may be raised. For example, Ara h 8 (peanut) and the major allergen Bet v 1 (Birch pollen) share common sequences. It is therefore possible that immunoglobulins are formed against one allergen that binds to the other allergen. In order to provide more clinically relevant results, the natural extract can be treated to remove or reduce the cross reactive antigenic components. If such cross reactive antigenic components are removed or reduced, it is preferred that these antigenic components are presented separately in the method or immunoassay. It will be understood that the antigenic components presented in the method are not necessarily those physically removed from the natural extract but can be prepared separately.

Cross reactions are frequently seen between certain types of pollen and foods. As an example, patients with birch pollen allergy may react with oral symptoms when consuming peanuts or other food sources containing structurally similar antigens - such symptoms include itching, swelling and reddening of the oral mucosa (often referred to as "oral allergy syndrome"). However, current understanding is that this will not lead to fatal or life-threatening allergy to peanut. Thus, without discrimination using depleted natural extract and the antigenic components in parallel, it would be harder to discriminate oral allergy syndrome to peanut (due to an allergy to birch pollen) with true allergy to peanut.

Examples of known cross reactive antigenic components include:
Mite allergens: Der p 1 and Der p 2, Food and pollen allergens: (PR10 proteins) Bet v 1, Cor a 1, Aln g 1, Mal d 1, Gly m 4, Api g 1 and Thaumatin-like proteins: Pru p 3 (peach), Zea m 14 (corn) (profilins) Bet v 2 and Phl p 12 with Sola t 8 (potato) and Api g 4 (celery), Furred animals: (lipocalins) Can f 6, Equ c 1, Fel d 4, (albumins) Can f 3 (dog), Equ c 2 (horse), Fel d 2 (cat).

It is a further preferred aspect of the invention that the natural extract is alternatively or additionally treated to remove other or further components. This would also result in a modified or depleted natural extract. For example, dog and cat dander may contain 50-80% albumin, which is bulky. If this component is removed, the rest of the natural extract remains, and this leaves greater binding capacity for the remaining components, some of which could include an epitope and be antigenic. Again, this increases the component resolution and the sensitivity.

In the invention, the natural extract is a modified natural extract, treated to remove known antigenic components, including cross reactive antigenic components and/or further components. In a preferred embodiment, at least one of the removed antigenic components is presented as a substance form in the methods or arrays of the invention. As explained previously, the separately presented antigenic components can be prepared recombinantly or isolated from the natural extract.

Components can be removed from a natural extract or preparation by any suitable means. For example, the extract can be filtered or other physical techniques of separation (e.g. affinity chromatography), alternatively, biological means of separation can be used (monoclonal antibodies, antigens or aptamers that bind to the specific component) and even chemical means of removing a component are suitable if the remaining components are unaffected. Furthermore, these techniques can also be used to isolate components from a substance. The isolated components can be used as a substance form.

It is an advantage of the present invention that known antigenic components can be removed from the natural extract. This allows data to be collected on the antigenicity of "minor" components in that natural extract without first having to identify them.

One or more natural extracts can be used in the immunoassay, methods, arrays or kits of the invention. It is envisaged that a plurality of natural extracts could be used. For example, an unmodified natural extract, simply isolated from the substance could be used, together with one or more modified natural extracts that have had one or more components removed. Different modified natural extracts could have different components removed in order to increase sensitivity by "enriching" minor components. Components that are removed could include known antigenic components or bulky components. This selective removal enhances the sensitivity of the assay.

The antigenic component of the substance can be isolated from the substance itself by methods known in the art (as discussed in relation to preparation of the modified natural extract), or can be produced recombinantly. Either natural or recombinant antigenic components can be used in the methods, arrays and kits of the invention. As mentioned previously, the antigenic components can be used individually, or can be combined to form mixed singular substance forms as desired. It is preferred that the antigenic components are used individually, allowing more data to be collected.

The antigenic component is preferably a known allergen. All of the allergens (antigenic components) described here have been named according to the standards set down by the WHO/IUIS Allergen Nomenclature Sub-committee (http://www.allergen.org/).

Figure 4 describes the basic principles of one aspect of the invention, and demonstrates the use of a plurality of substance forms in one assay format. Given a substance in its natural form, the composition can be altered by selectively removing one or more of its components (e.g. antigen 1, antigen 2 and antigen 3) to enrich the content of some or all the remaining antigenic components (e.g. unknown or minor antigens) of said substance. The unmodified natural extract and/or the modified or depleted version are immobilised together with the single or combined antigenic components of said substance to increase the binding capacity of the immunoassay or method. The increase in the binding capacity is then translated into higher assay sensitivity. The data may be interpreted by an algorithm. Indeed, an algorithm may look for the highest fluorescence signal, which may derive from either a single substance form (e.g. natural extract or a single/combined antigenic component) or a combination of them (e.g. combined data from the Natural extract(s) and/or single/combined antigenic components). The scenarios presented demonstrate what happens if an individual has immunoglobulins specific for major, minor and/or unknown antigens. It is the use of a plurality of substance forms that increases the sensitivity of testing, since several of these individuals would be missed if only single components or natural extract alone were used in an assay.

In a third aspect, the present invention relates to a method for enhancing the binding capacity of a substance, comprising the selective removal of one or more components from the substance. The binding capacity relates to the ability of substance-specific immunoglobulin or other specifically binding molecules to bind to the substance.

In this aspect of the invention, the substance is usually a natural extract, prepared as described herein. The components selected for removal can be known antigenic components, including cross reactive antigenic components or common or bulky components. Methods have been discussed herein for methods to enable the selective removal of components.

The modified natural extract is used in the present invention.

Where the present invention describes the use of an antigen, this can be substituted by the term allergen. It is a preferred aspect that the substance is allergenic.

The term "plurality" as used herein is defined as two, or more than two.

In a fourth aspect, there is provided an array comprising a plurality of substance forms provided at discrete locations, wherein at least one form is a natural extract, modified by removal of one or more antigenic components. In a preferred embodiment, the array also comprises one or more antigenic components of the substance, provided at discrete locations.

In a fifth aspect, there is provided a kit comprising an array which comprises a plurality of substance forms provided at discrete locations, wherein at least one form is a natural extract, modified by removal of one or more antigenic components, and one or more binding members. The array can be as described previously. The binding members can be detection antibodies.

It is preferred that the array is a microarray. The array can take any form suitable for use as an immunoassay. The array is essentially an arrangement of substance forms bound to a solid surface, such that the location of each form is known. The array can include multiple different substances, such that multiple detection steps are carried out in parallel. Thus, the most preferred format for the array is a multiplexing assay.

According to several aspects of the invention, the formation of an immune-complex between a substance form and a substance-specific immunoglobulin is detected. This detection may be via any suitable means known to those skilled in the art, or as discussed herein.

The formation of an immune-complex demonstrates that the immunoglobulin has reacted with the substance. The level of immune-complex formation is thus indicative of the level of the reactivity of the substance-specific immunoglobulin, and can be used to quantify this reactivity.

The levels of immunoglobulin reactivity are thus determined by contacting a sample (which may comprise specific immunoglobulin) with a plurality of antigen forms substantially in parallel (or substantially simultaneously) and determining the amount of each specific immunoglobulin from the sample that is bound to each substance form.

Preferably the levels of immunoglobulin reactivity to a substance form (or formation of an immune complex) is measured by means of an immunoassay using an anti-immunoglobulin antibody (detection antibody). Generally, the detection antibodies (or fragments and parts thereof) used in an immunoassay are specific for a certain isotype of immunoglobulin. For example, it is possible to detect only IgG, IgM, IgA, IgD or IgE. This is useful, since each of the isotypes may have a different clinical significance.

When the immunoassay is concerned with the diagnosis of allergy, it is useful to detect only IgE immunoglobulin, since they mediate allergy. Anti-lgE antibodies react with the IgE isotype of human immunoglobulin. Therefore, in certain embodiments the presence or amount of IgE bound to each antigen is determined or quantified using an anti-lgE antibody.

The detection antibody may be a polyclonal, monoclonal, bispecific, humanised or chimeric antibody although affinity-purified antibodies and yet more particularly monoclonal antibodies may generally be preferred. Such detection antibodies may be conventional or recombinant antibodies and may consist of a single chain but would preferably consist of at least a light chain or a heavy chain. However, it will be appreciated that at least one complementarity determining region (CDR) is required in order to bind a target such as an antigen to which the antibody has binding specificity.

Methods of making detection antibodies that bind to an isotype of an immunoglobulin are known in the art. For example, if polyclonal antibodies are desired, then a selected mammal, such as a human, mouse, rabbit, pig, sheep, camel, goat or horse may be immunised with the antigen of choice, such as a heterologous IgE, IgA or IgG. The serum from the immunised animal is then collected and treated to obtain the antibody, for instance by immunoaffinity chromatography.

Monoclonal detection antibodies may be produced by methods known in the art. The general methodology for making monoclonal antibodies using hybridoma technology is well known (see, for example, Kohler, G. and Milstein, C, Nature 256: 495-497 (1975); Kozbor et al, Immunology Today 4: 72 (1983); Cole et al, 77-96 in Monoclonal Antibodies and Cancer Therapy, Alan R. Liss, Inc. (1985).

A detection antibody, as referred to herein, should consist of an epitope-binding region, such as CDR. The antibody may be of any suitable class, including IgE, IgM, IgD, IgA and, in particular, IgG. The various subclasses of these antibodies are also envisaged. In particular embodiments, fragments of a detection antibody or polypeptides derived from such an antibody which retains the binding specificity of the detection antibody may be used. Such fragments include, but are not limited to antibody fragments, such as Fab, Fab', F(ab')2 and Fv, all of which are capable of binding to an epitope.

The term "detection antibody" also extends to any of the various natural and artificial antibodies and antibody-derived proteins which are available, and their derivatives, e.g. including without limitation polyclonal antibodies, monoclonal antibodies, chimeric antibodies, humanized antibodies, human antibodies, single-domain antibodies, whole antibodies, antibody fragments such as F(ab')2 and F(ab) fragments, Fv fragments (noncovalent heterodimers), single-chain antibodies such as single chain Fv molecules (scFv), minibodies, oligobodies, dimeric or trimeric antibody fragments or constructs, affibodies or other forms of binding molecules, etc. The term "detection antibody" does not imply any particular origin, and includes antibodies obtained through non-conventional processes, such as phage display. Antibodies of the invention can be of any isotype (e.g. IgA, IgG, IgM) and may have a κ (kappa) or a λ (lambda) light chain.

Whilst it is preferred that the binding member for the immunoglobulin is an antibody or derivative thereof, it is also envisaged that any suitable binding member could be used. Thus, the binding member could be an aptamer or suchlike. Aptamers are oligonucleic acid (such as DNA or RNA) or peptide molecules that bind to a specific target molecule. Suitable binding members are well known in the art. Thus, the binding member can be any suitable molecule which can bind to immunoglobulin.

The invention therefore extends to the use of binding members or molecules, detection antibodies and binding fragments which have binding specificity to an immunoglobulin isotype for use in the present invention.

It is preferred that the detection antibody is an anti- IgE antibody.

The term "specifically binds" or "binding specificity" refers to the ability of a binding member, an antibody or fragment thereof to bind to a target with a greater affinity than it binds to a non-target epitope. For example, the binding of an antibody to a target epitope may result in a binding affinity which is at least 10, 50, 100, 250, 500, or 1000 times greater than the binding affinity for a non-target epitope. In certain embodiments, binding affinity is determined by an affinity ELISA assay. In alternative embodiments, affinity is determined by a BIAcore assay. Alternatively, binding affinity may be determined by a kinetic method.

In particular embodiments the detection antibody is a labelled antibody, or the binding member is labelled. By way of non-limiting example labelling may be by conjugation to an enzyme such as a peroxidase or a chemiluminescent or fluorescent compound, such as Alexa Fluor 555 or a mass tag. Any suitable label is envisaged by the present inventors.

In other embodiments the detection antibody is unlabelled and is detected using a further antibody, commonly called a secondary antibody, which may be labelled as described. The same also applies to binding members, where a second binding member can be used to detect the first.

In a preferred embodiment, the detection antibody is an unlabelled mouse monoclonal antibody directed against an immunoglobulin such as IgE and which is detected using a labelled secondary antibody, such as an anti-mouse IgG. In certain embodiments, one or more luminescent or fluorescent moieties may be bound to avidin/streptavidin, which in turn may be bound to biotin chemically conjugated to an antibody. In certain further embodiments, lectins (Protein A/G/L) can be linked to a luminescent or fluorescent molecule which may also be attached to an antibody or other protein conjugate. In preferred embodiments a tyramide signal amplification system is utilised that uses the catalytic activity of horseradish peroxidase (HRP) to generate high-density labelling of an antibody. Suitable labels and labelling methods are known in the art and would be apparent to the skilled artisan.

In particular embodiments the labelled antibody comprises a fluorescent label.

Appropriate fluorescent labels are well known in the art, and can include, by way of non-limiting example, Alexa Fluor 488, Alexa Fluor 555, R-phycoerythrin, Aqua, Texas-Red, FITC, rhodamine, a rhodamine derivative, fluorescein, a fluorescein derivative, cascade blue, Cy5 or Cy3.

The detection method may be by any suitable method known in the art such as by optical detection including fluorescence measurement, colourimetry, flow cytometry, chemiluminescence and the like. Other methods include electrochemical, radioactive, piezoelectric methods and the like. Yet other methods of detection of binding may be by surface Plasmon resonance (SPR), surface Plasmon microscopy (SPM), surface Plasmon fluorescence spectroscopy or SELDI mass spectroscopy and the like. The skilled person will appreciate that detection may be performed using a combination of detection methods.

Particularly, detection of binding is by measurement/detection of a luminescent signal, for example, chemiluminescent light produced by a chemiluminescent compound.

The solid support may be any material known in the art, for example, a glass carrier, synthetic carrier, silicon wafer or membrane. Suitable materials include plastics, glasses, silicon, ceramics or organic polymers including polystyrene, polycarbonate, polypropylene, polyethylene, cellulose and nitrocellulose. The surface itself may be in the form, or part, of a slide, sheet, microplate or microtitre plate, tray, membrane, fibre, well, pellet, rod, stick, tube, bead and the like.

Use of the term "bound" is intended to mean that a substance is retained, immobilised or substantially attached to a surface at the molecular level (i.e., through a covalent or noncovalent bond or interaction). The immobilisation method used should be reproducible, applicable to antigens of different properties (size, hydrophilic, hydrophobic), amenable to high throughput and automation, and maintain the ability of the antigen to be form a complex with an antibody. By way of non-limiting example, suitable methods known in the art include passive adsorption, affinity based binding, covalent coupling to chemically activated surfaces, photochemical cross-coupling and the like.

The term "address" is used to refer to a distinct feature of a solid support or defined location on a solid support that allows a specific antigen to be identified enabling the level of immunoglobulin reactivity to that specific antigen to be determined.

In one embodiment the solid support is a plurality of beads each being separately identifiable by means of an address. Suitable addresses include RFID tags, mass tags, fluorescent tags, optical encoding, digital magnetic tags, spectrometric encoding and the like.

In other embodiments the solid support is a microarray. The term "microarray" as used herein, refers to an ordered array of spots presented for binding of immunoglobulins. Microarrays of the present invention comprise at least two, at least nine, at least fifty, at least one hundred, at least five hundred or at least one thousand spots. In some embodiments the microarray may comprise at least 10,000, 40,000, 100,000, or 1,000,000 different and distinct spots. The spots may be at a density of from about 100/cm2 to about 1000/cm2 or greater.

A "spot" refers to a reagent or reagents, in this instance a specific substance form, deposited at a particular address, in this instance a physical location, on the array surface. Typically a spot is characterised by the presence of one or more specific molecules (e.g. particular proteins, allergen extracts, antigens, etc.). Spots may be from 10 to 2000 µm in diameter, 50-500 µm in diameter or 200-400 µm in diameter. Substance forms may be applied to the surface of a solid support at a spotting concentration of from 0.001 to 10mg/ml, preferably 0.008mg/ml to 3mg/ml in order to form an array.

Systems suitable for measuring or reading fluorescence signals from microarrays are known. Generally an image is constructed by scanning the slide in two dimensions under a laser spot. An image can be acquired in about one minute, but the analysis is complicated in terms of the image analysis processes. These processes can be complex because of both the large amount of data generated and the analysis algorithms required to produce an unambiguous measurement of the integrated signal from each spot or micro-spot.

In their previous patent application published as WO2003/091712, the inventors realised that it is possible to read the fluorescence by illuminating the entirety of each spot on an array and taking a measurement of the fluorescence of the entire spot in a single measurement rather than scanning across and illuminating a fraction of each spot several times in order to build up an image of each spot pixel by pixel. This approach enables LEDs, which are low cost light sources, to be used as the illumination source. Each fluorescent molecule receives the same optical energy as it would do if a coherent light source was used as the illumination source. However, the detector yields a single reading requiring no further signal analysis (rather than a 400 pixel image per spot which requires complicated image processing to calculate an overall measurement). Use of a coherent light source may in some circumstances be a disadvantage, because of additional noise introduced in the signal arising from the interference effects.

In other embodiments the use of microcantilevers is envisaged as disclosed in International Patent publication WO2006/138161. In yet other embodiments the assay is carried out using one or more microfluidic chips.

In the present invention, the sample is contacted with a plurality of forms of a substance, including the natural extract, modified by removal of one or more antigenic components. The formation (or indeed lack thereof) of an immune-complex is detected for each of the forms. The reactivity of immunoglobulins to each of the forms is thus determined.

In order to determine whether a sample contains substance-specific immunoglobulins are present in a sample, the level of substance-specific immunoglobulin is examined for each substance form. The level of substance-specific immunoglobulin may be represented by the signal from the label, as depicted in figure 1. In one embodiment, the immunoglobulin level for each form is combined for a particular substance, and the cumulative result is taken. This need not be for all forms of the substance present, but could be for a selected subset (for example 2, 3, 4, 5, 6, 7, 8 or 9 substance forms). For example, the levels only for the antigenic components could be combined. In an alternative embodiment, the form with the highest level of substance-specific immunoglobulin is taken to represent whether or not the sample contains substance specific immunoglobulins. In a further alternative embodiment, a statistical analysis is undertaken using all of the data for the substance forms. Suitable algorithms are discussed further below.

The level of substance specific immunoglobulin (the level of data, signal or result) may be provided as a numerical value, such as a class score (e.g. from 0 to 6) or antibody concentration (kU/L). In this instance, it is possible to proceed with a statistical analysis. Alternatively, the data may be analysed simply as the presence or absence of an immune complex if a signal goes beyond a predetermined threshold. Any measure of immunoglobulin reactivity or immune complex formation can be provided as the data or results from the immunoassay.

Once the results from all of the forms have been collected and analysed, it can be determined if the sample contains substance-specific immunoglobulins. This is particularly relevant for the aspects of the invention that relate to methods of determining the presence of immune complexes and/or diagnosis.

Whilst the reactivity of immunoglobulins in the sample to each of the substance form may be determined, the analysis of the results can be varied. Data for each of the substance forms can be read. Alternatively, the highest result, such as class score or antibody/immunoglobulin concentration can be taken for each substance, when all of the substance forms are compared. This highest result can be taken to indicate the presence of substance-specific immunoglobulins. Alternatively, the data from selected substance forms can be combined. For example, the data from a depleted natural extract and one or more of the removed antigenic component can be combined.

Alternatively, the data from one or more forms can be combined or summed in order to determine the presence of substance-specific immunoglobulins. Further analysis can also be performed. For example, if more than one known cross-reactive antigenic components are presented, the data for such components can be combined to provide information on the cross reactive status. Alternatively, data from more than one major antigenic component can be combined in order to give an indication of severity.

It is envisaged that the data from each of the substance forms is analysed automatically, using an algorithm. Any suitable algorithm can be used. It is an aim that the algorithm analyses the data and provides a single read out to state whether or not substance specific immunoglobulins are present in the sample. Thus, a positive or negative result is desired. Such a format makes this a desirable point-of-care method, but the invention is not so limited, and could be performed at a remote location to the individual.

The entire method can be automated, and a suitable instrument for this is the Microtest instrument from the present applicants (www.microtestdx.com). Suitable software can be included to analyse the plurality of results and provide a single positive or negative result.

However, it will be appreciated that the method also provides information on the detailed antigen-specific immunoglobulins, and this data can be extracted separately, if it is desired to know which antigenic components are involved.

One embodiment of this invention involves testing an individual for their substance specific immunoglobulin status, be this for allergy or diagnosis of an autoimmune disease.

The immunoassay is performed on a sample taken from an individual, who is suspected of having one or more immunoglobulins to one or more specific substances. For example, the individual may have allergic symptoms that indicate the presence of immunoglobulins to allergens. Alternatively the individual may have immunoglobulins to self substances (also known as autoantibodies) and these can be detected using the method of the invention. Furthermore, the individual may have a viral, bacterial, parasitic or fungal infection (either current or in the past) to which immunoglobulins are raised, and the present immunoassay can be used to determine the status of these immunoglobulins, and thus the vaccination and/ or infection history of an individual, or the cause of a present infection.

In a sixth aspect, there is provided a method to determine the substance - specific immunoglobulin status of an individual comprising contacting a sample from said individual substantially simultaneously with a plurality of forms of said substance, wherein at least one form is a natural extract, modified by removal of one or more antigenic components, and determining whether an immune complex is formed.

In a preferred embodiment, antigenic component specific immunoglobulin status is also determined.

The immunoglobulin mentioned in any of the methods or assays of the invention can be any suitable immunoglobulin, but it is preferred that the immunoglobulin is Immunoglobulin E. It is preferred that the substance is allergenic.

In a seventh aspect, there is provided a method for the diagnosis of an allergy to a substance in an individual, said method comprising contacting a sample from said individual substantially simultaneously with a plurality of forms of a substance, wherein at least one form is a natural extract, modified by removal of one or more antigenic components, and the presence of an immune complex with one or more of the forms indicates the presence of an allergy to that substance.

For any aspect of the invention, the presence of an immune complex with each form of the invention is detected. The presence of one or more immune complexes can be sufficient to make a diagnosis or determine the presence of substance-specific immunoglobulins. However, the data from these methods can also be analysed as described previously. Thus, in order to make a determination or diagnosis, the highest signal can be taken as an indication of a positive result, or alternatively, the mean reading for all forms can be taken. The reading for each form will give information on the specific allergy profile for the individual from which the sample was taken.

The invention will now be described in more detail, with reference to the Examples:

### Example 1:

40 plasma samples were obtained from individuals.

These samples were exposed to a plurality of forms of the dust mite allergen. These forms were: natural extract (D01) and the dust mite major components: Der p 1 and Der p 2.

Each form was immobilised onto a pre-treated microarray slide in an ordered matrix.

The presence/absence of dust mite specific-lgE was determined by a proprietary fluorometric enzyme immunoassay designed to determine the IgE response to a range of specific allergens, as described below. Materials are available from Microtest Diagnostics (www.microtestdx.com)

The first step of the assay is the addition of a sample onto the chip through the reagent port of the Microtest Biochip. The fluid flows into the chamber and each plurality of forms of the allergen (e.g. dust mite) reacts with the antigen-specific IgE contained in the sample. Following 60 minutes incubation the Microtest machine flushes the sample away and adds the secondary antibody (anti-human IgE), which binds with the allergen-lgE complex. Then, after 45 minutes incubation, the Microtest machine flushes the secondary antibody away and adds the tertiary antibody, an enzyme labelled antibody that detects the allergen-antibody complexes. After 45 minutes incubation the Microtest machine flushes the unbound tertiary antibody away and adds an amplification buffer, which is used to develop the fluorescence. Finally, after 20 minutes incubation the Microtest machine flushes the amplification buffer away. Then, using a novel and inexpensive solution for capturing and elaborating the fluorescent signal, the machine analyses the miniaturized array by reading the fluorescence of the allergen-lgE immunocomplex.

The results (Figures 1, 2 and 3, and tables 1 and 2) demonstrate that the invention has improved the sensitivity of the system. The highest reading value obtained from either the of the sum of the fluorescence of Der p 1 and Der p 2 or the natural extract reactivity is equal or higher than the single fluorescent signals obtained either the natural extract or single component readings (i.e. standard prior art method). Indeed, during the fluorescence analysis an internal algorithm will look for the best result (highest fluorescence signal), which may derive from either the single antigen/allergen (e.g. natural extract, D01) or a combination of them (e.g. dust mite components: Der p 1 + Der p 2).

The low fluorescence reactivity of some of the samples with high specific-lgE level (e.g. ^{∼}50 and ^{∼}100 kU/L) plotted in Figure 3 may be explained by the absence of other *Dermatophagoides pteronyssinus* antigens, that together with Der p 1 and Der p 2, are the major allergen components for the dust mite.

**Table 1: Shows the numerical values used to create the bar chart in Figure 1.**

| **Class Score** | **D01- Extract** | **Der p1** | **Der p2** | **Dp1 + Dp2** | **Max value (D01 or Dp1 + Dp2)** |
|---|---|---|---|---|---|
| **0-1** | 115 | 508 | 96 | 605 | 605 |
| **2** | 1685 | 2369 | 101 | 2469 | 3766 |
| **3** | 844 | 10260 | 9824 | 20084 | 20084 |
| **4** | 975 | 19244 | 11965 | 31209 | 31209 |
| **5** | 13705 | 26602 | 24816 | 51419 | 55962 |

**Table 2: Shows the correlation between allergen-specific immunoglobulin concentration and Class score**

| **SPECIFIC IgE TEST LEGEND** | |
|---|---|
| **kU/L** | **CLASS SCORE** |
| < 0.35 | 0 |
| 0.35 - 0.70 | 1 |
| 0.71 - 3.50 | 2 |
| 3.51 - 17.5 | 3 |
| 17.6 - 50.0 | 4 |
| 50.1 - 100 | 5 |
| > 100 | 6 |

Figure 5 depicts 5 selected samples from the 40 analysed. Sample A shows the highest immune complex formation with the natural extract D01. Sample B shows the highest immune complex formation with Der p 1, whereas sample C the highest levels are with Der p 2. Sample D shows that it is the combination of results from Der p 1 and Der p 2 that gives the highest result, and sample E demonstrates that minor/unknown antigen in the natural extract cause the highest level of immune complex formation. Using only 1 or more individual components or the natural extract in isolation would not have led to an accurate result for these samples. The specific data for this figure is presented in table 3.

**Table 3:**

| **Samples ID_kU/L** | **D01 - Extract** | **Der p1** | **Der p 2** | **Dp1 + Dp2** |
|---|---|---|---|---|
| Sample A_1.62 | 6568 | 641 | 0 | 641 |
| Sample B_4.76 | 136 | 15920 | 581 | 16501 |
| Sample C_12.25 | 280 | 774 | 23914 | 24688 |
| Sample D_20.50 | 975 | 19244 | 11965 | 31209 |
| Sample E_100.00 | 51347 | 9444 | 1014 | 10458 |

### Example 2

### Preparation of a depleted natural extract of peanut

Natural peanut extract was prepared by collecting raw peanuts, which were blended into a paste and then solubilised into a liquid form using standard techniques, such as those described in Hefle et al, The Journal of Allergy and Clinical Immunology, Volume 95, Issue 4, Pages 837-842, April 1995. This resulted in natural extract of peanut.

Rabbit polyclonal antibodies to Ara h 8 can be raised by standard techniques (such as those described in Wen et al, Anal Bioanal Chem. 2005 Jul; 382(5):1217-26 in relation to rabbit polyclonal antibodies to Ara h 1.) These antibodies were covalently linked/coupled to a matrix for use in a chromatography column.

This natural peanut extract was loaded onto the chromatography column containing antibodies directed to Ara h 8, an antigenic component. As the natural peanut extract passes through the column, the antibodies selectively bound to the Ara h 8 present in the extract. The remaining extract is untouched and flowed though the column, and collected.

A suitable method is described in Sen et al, The Journal of Immunology July 15, 2002 vol. 169 no. 2 882-887, however, in this instance the natural extract is discarded after it flows through the column since it was an aim to look at the proteins removed from the natural extract.

The resulting liquid extract collected from the column was natural extract of peanut depleted of Ara h 8.

It should be noted that applying a salt gradient to the column once the natural extract has been eluted and collected will allow for collection of Ara h 8, which can be used as a different form of peanut in an immunoassay.

Ara h 8 is a known cross-reactive antigenic component.

## Claims

1. A method for detecting the presence or absence of substance-specific immunoglobulin in a sample, said method comprising contacting said sample with a plurality of forms of said substance substantially simultaneously and determining for each form separately whether an immune-complex is formed, wherein at least one form is a natural extract, modified by removal of one or more antigenic components.

2. A method as claimed in claim 1 wherein said method further comprises the step of quantifying the level of substance-specific immunoglobulin, optionally wherein the level of substance-specific immunoglobulin is provided as a class score or immunoglobulin concentration.

3. A method as claimed in claim 2 wherein the level of substance-specific immunoglobulin is determined for each of the forms of substance.

4. A method as claimed in claim 2 or 3 wherein the levels of substance-specific immunoglobulin are combined for 2 or more of the forms of the substance.

5. A method as claimed in any preceding claim wherein said method allows the substance-specific immunoglobulin status of an individual to be determined.

6. A method as claimed in any preceding claim wherein said method includes a step of diagnosing an allergy to a substance in an individual, and the presence of an immune complex with one or more forms indicates sensitisation to that substance.

7. A method as claimed in any preceding claim wherein the modified natural extract is prepared by the selective removal of one or more components from the natural extract, preferably by component-specific affinity depletion.

8. A method as claimed in claim 1 wherein said antigenic component is a cross reactive component.

9. A method as claimed in any preceding claim, wherein the one or more removed components are separately presented as one or more forms of the substance.

10. A method as claimed in claim 9 wherein the levels of substance-specific immunoglobulin is determined for the modified natural extract and one or more of the removed components and are then combined.

11. A method as claimed in any preceding claim wherein at least one form of said substance is an antigenic component or a combination of antigenic components.

12. An array comprising a plurality of forms of a substance provided at discrete locations, wherein at least one form is a natural extract modified by removal of one or more antigenic components, and at least one form is an antigenic component or a combination of antigenic components.

13. A kit comprising an array as claimed in claim 12, and one or more detection molecules, optionally wherein the detection molecules are antibodies or derivatives thereof.

14. A method as claimed in any one of claims 1 to 11 wherein said immunoglobulin is Immunoglobulin E or Immunoglobulin G.

15. A method as claimed in any one of claims 1 to 11 wherein said forms are immobilised separately on a solid support, optionally in a microarray format.

## Patentansprüche

1. Verfahren zum Detektieren des Vorhandenseins oder Nichtvorhandenseins von substanzspezifischem Immunglobulin in einer Probe, das Verfahren umfassend ein Inkontaktbringen der Probe mit einer Vielzahl von Formen der Substanz im Wesentlichen gleichzeitig und Bestimmen separat für jede Form, ob ein Immunkomplex gebildet ist, wobei mindestens eine Form ein natürlicher Extrakt ist, der durch Entfernen einer oder mehrerer antigener Komponenten modifiziert ist.

2. Verfahren nach Anspruch 1, wobei das Verfahren ferner den Schritt eines Quantifizierens des Gehalts an substanzspezifischem Immunglobulin umfasst, optional wobei der Gehalt an substanzspezifischem Immunglobulin als Klassenpunktzahl oder Immunglobulinkonzentration bereitgestellt ist.

3. Verfahren nach Anspruch 2, wobei der Gehalt an substanzspezifischem Immunglobulin für jede der Substanzformen bestimmt wird.

4. Verfahren nach Anspruch 2 oder 3, wobei die Mengen an substanzspezifischem Immunglobulin für 2 oder mehrere der Formen der Substanz kombiniert sind.

5. Verfahren nach einem der vorherigen Ansprüche, wobei das Verfahren eine Bestimmung des substanzspezifischen Immunglobulinstatus eines Individuums ermöglicht.

6. Verfahren nach einem der vorigen Ansprüche, wobei das Verfahren einen Schritt zum Diagnostizieren einer Allergie gegen eine Substanz bei einem Individuum beinhaltet und das Vorhandensein eines Immunkomplexes mit einer oder mehreren Formen eine Sensibilisierung auf diese Substanz angibt.

7. Verfahren nach einem der vorherigen Ansprüche, wobei der modifizierte natürliche Extrakt durch die selektive Entfernung einer oder mehrerer Komponenten aus dem natürlichen Extrakt hergestellt wird, vorzugsweise durch komponentenspezifische Affinitätsverarmung.

8. Verfahren nach Anspruch 1, wobei die antigene Komponente eine kreuzreaktive Komponente ist.

9. Verfahren nach einem der vorherigen Ansprüche, wobei die eine oder die mehreren entfernten Komponenten separat als eine oder mehrere Formen der Substanz präsentiert werden.

10. Verfahren nach Anspruch 9, wobei der Gehalt an substanzspezifischem Immunglobulin für den modifizierten natürlichen Extrakt und eine oder mehrere der entfernten Komponenten bestimmt und diese dann kombiniert werden.

11. Verfahren nach einem der vorherigen Ansprüche, wobei mindestens eine Form der Substanz eine antigene Komponente oder eine Kombination von antigenen Komponenten ist.

12. Anordnung, umfassend mehrere Formen einer Substanz, die an einzelnen Stellen bereitgestellt sind, wobei mindestens eine Form ein natürlicher Extrakt ist, der durch Entfernen einer oder mehrerer antigener Komponenten modifiziert ist, und mindestens eine Form eine antigene Komponente oder eine Kombination von antigenen Komponenten ist.

13. Kit, umfassend ein Array nach Anspruch 12 und ein oder mehrere Detektionsmoleküle, optional wobei die Detektionsmoleküle Antikörper oder Derivate davon sind.

14. Verfahren nach einem der Ansprüche 1 bis 11, wobei das Immunglobulin Immunglobulin E oder Immunglobulin G ist.

15. Verfahren nach einem der Ansprüche 1 bis 11, wobei die Formen separat auf einem festen Träger immobilisiert sind, optional in einem Microarray-Format.

## Revendications

1. Procédé de détection de la présence ou de l'absence d'immunoglobuline spécifique à une substance dans un échantillon, ledit procédé comprenant la mise en contact dudit échantillon avec une pluralité de formes de ladite substance sensiblement de manière simultanée et le fait de déterminer pour chaque forme de manière séparée si un complexe immun est formé, dans lequel au moins une forme est un extrait naturel, modifié par le retrait d'au moins un composant antigénique.

2. Procédé selon la revendication 1, dans lequel ledit procédé comprend en outre l'étape de quantification du niveau d'immunoglobuline spécifique à une substance, dans lequel le niveau d'immunoglobuline spécifique à une substance est éventuellement fourni en tant que score de classe ou concentration en immunoglobuline.

3. Procédé selon la revendication 2, dans lequel le niveau d'immunoglobuline spécifique à une substance est déterminé pour chacune des formes de substance.

4. Procédé selon la revendication 2 ou la revendication 3, dans lequel les niveaux d'immunoglobuline spécifique à une substance sont combinés pour au moins deux des formes de la substance.

5. Procédé selon l'une quelconque des revendications précédentes, dans lequel ledit procédé permet de déterminer le statut d'immunoglobuline spécifique à une substance d'un individu.

6. Procédé selon l'une quelconque des revendications précédentes, dans lequel ledit procédé comprend une étape de diagnostic d'une allergie à une substance chez un individu, et la présence d'un complexe immun avec au moins une forme indique une sensibilisation à cette substance.

7. Procédé selon l'une quelconque des revendications précédentes, dans lequel l'extrait naturel modifié est préparé par le retrait sélectif d'au moins un composant de l'extrait naturel, de préférence par l'appauvrissement d'affinité spécifique à un composant.

8. Procédé selon la revendication 1, dans lequel ledit composant antigénique est un composant à réactivité croisée.

9. Procédé selon l'une quelconque des revendications précédentes, dans lequel l'au moins un composant retiré est présenté de manière séparée en tant qu'au moins une forme de la substance.

10. Procédé selon la revendication 9, dans lequel les niveaux d'immunoglobuline spécifique à une substance sont déterminés pour l'extrait naturel modifié et pour au moins un des composants retirés et sont ensuite combinés.

11. Procédé selon l'une quelconque des revendications précédentes, dans lequel au moins une forme de ladite substance est un composant antigénique ou une combinaison de composants antigéniques.

12. Réseau comprenant une pluralité de formes d'une substance apportée à des emplacements distincts, dans lequel au moins une forme est un extrait naturel modifié par le retrait d'au moins un composant antigénique, et au moins une forme est un composant antigénique ou une combinaison de composants antigéniques.

13. Kit comprenant un réseau tel que revendiqué en revendication 12, et au moins une molécule de détection, les molécules de détection étant éventuellement des anticorps ou des dérivés de celles-ci.

14. Procédé selon l'une quelconque des revendications 1 à 11, dans lequel ladite immunoglobuline est l'immunoglobuline E ou l'immunoglobuline G.

15. Procédé selon l'une quelconque des revendications 1 à 11, dans lequel lesdites formes sont immobilisées de manière séparée sur un support solide, éventuellement dans un format de micro-réseau.
